# EUROPEAN PATENT APPLICATION

(11) **EP 4 570 258 A1**
(43) Date of publication of application: **18.06.2025**
(21) Application number: 24218844.9
(22) Date of filing: 10.12.2024
(51) Int. Cl.: A61K 35/19, A61M 1/02, A61M 1/00, C12N 5/00, A61K 9/19, A61P 39/00

(54) **USE OF A PURIFIED EXOSOME DRY POWDER FOR RELIEVING INFLAMMATION OR INJURY**

(30) Priority: 11.12.2023 US 202363608323 P
(71) Applicant: Spirit Scientific Co., Ltd., New Taipei City 22175 (TW); Lin, Dao Lung, Steven, New Taipei City 22175 (TW)
(72) Inventor: CHEN, Chin-Ho, 22175 New Taipei City (TW); LIN, Dao Lung, 22175 New Taipei City (TW)
(74) Representative: Haseltine Lake Kempner LLP

(57) **Abstract**

The present invention provides a use of a purified platelet-derived exosome dry powder for relieving inflammation or injury, wherein the number of platelet-derived exosomes in each gram of the purified platelet-derived exosome dry powder is more than or equal to 1×10¹⁰.

## Description

### [FIELD OF THE INVENTION]

The present invention relates to a purified platelet-derived exosome dry powder, and more particular to a use of the purified platelet-derived exosome dry powder for relieving inflammation or injury.

### [BACKGROUND OF THE INVENTION]

Inflammation is the body's defense mechanism. Inflammatory reactions are often triggered in various parts of the body due to infections, traumas or hypersensitivity in order to remove harmful irritants and pathogens to promote tissue repairing. Inflammation can be divided into acute inflammation and chronic inflammation such as dermatitis, allergic rhinitis, pneumonia, hepatitis and enteritis. Dermatitis can be divided into irritant contact dermatitis (ICD) and allergic contact dermatitis; severe pneumonia can lead to disease such as chronic obstructive pulmonary disease (COPD) and pulmonary fibrosis; while severe hepatitis can lead to disease such as liver cirrhosis and cancer.

Fibroblasts play an important role in the repair process of various tissues throughout the body. During the repair process after tissue injury (usually accompanied by an inflammatory response), a series of wound repair responses occur, including cell proliferation, cell migration, fibrogenesis, resolution, and remodeling (Reference 1). Taking skin wounds as an example, fibroblasts will quickly migrate to the provisional matrix and proliferate rapidly, causing fibrogenesis in the injured area. Moreover, fibroblasts are the main source of extracellular matrix (ECM) protein, including collagen and fibronectin, which can form granulation tissue and provide structural integrity for wounds.

Exosomes are nanoscale extracellular vesicles (EVs) with a diameter of approximately 30-200 nanometers, secreted by most eukaryotic cells, and contain bioactive molecules such as proteins, RNA, DNA, microRNAs, and metabolites. Therefore, exosomes play a key role in intercellular communication by transmitting different information between cells through the bioactive molecules they carry (References 2, 3, 4). Exosomes are present in various biological fluids, including plasma, urine, semen, saliva, bronchial fluid, cerebrospinal fluid (CSF), breast milk, serum, amniotic fluid, synovial fluid, tears, lymph, bile, and gastric acid (Reference 3). Plasma and serum contain exosomes derived from white blood cells, red blood cells, and other cells.

Current studies have found that exosomes derived from certain cells or tissues can achieve the purpose of tissue regeneration and repair by inhibiting inflammatory responses, promoting proliferation, inhibiting apoptosis, and promoting angiogenesis. However, clinical studies have found that exosomes are prone to rupture, have unstable quality, and are difficult to preserve during the separation and purification process, which in turn affects the therapeutic effect.

There is an urgent need for a pharmaceutical composition that overcomes current technological limitations to relieve or treat inflammation or injury in various parts of the body.

### [Summary of The Invention]

In view of the deficiencies in the prior art, the objective of the present invention is to provide a purified platelet-derived exosome dry powder that effectively relieves or treats inflammation or injury.

Another objective of the present invention is to provide a method for preparing the purified platelet-derived exosome dry powder.

A further objective of the present invention is to provide the purified platelet-derived exosome dry powder for relieving or treating inflammation or injury.

A further objective of the present invention is to provide the preparation of a pharmaceutical composition or health composition for use in relieving or treating a degree of inflammation or injury.

The present invention provides a method for preparing a purified platelet-derived exosome dry powder, which comprises: (a) taking one unit of a solution derived from blood and containing platelets, and performing a process of purifying platelets to obtain a pure platelet solution; wherein step (a) comprises: (a1) taking one unit of a solution derived from blood and containing platelets; (a2) performing a first sedimentation process to separate the solution derived from blood and containing platelets into a plasma layer and a blood cell layer; (a3) removing the blood cell layer to obtain the plasma layer solution; (a4) performing a second sedimentation process to cause the platelets to settle and form a pellet; (a5) removing the supernatant and mixing the pellet with a solvent to obtain the pure platelet solution; (b) performing an activation procedure to activate platelets in the pure platelet solution and release exosomes to obtain an activated pure platelet solution, wherein the activation procedure is a co-treatment procedure of collagen and thrombin; (c) performing a purification process, including: (c1) performing a centrifugation process to remove most of the platelet-associated structures, i.e., removing the pellet obtained after centrifugation; (c2) performing a filter membrane filtration process to completely remove platelet-associated structures, obtaining a purified platelet-derived exosome solution; and (d) performing a drying process on the purified platelet-derived exosome solution to obtain a purified platelet-derived exosome dry powder containing a plurality of platelet-derived exosomes.

In some embodiments, the drying process is a freeze-drying process.

In some embodiments, step (a3) involves a process of removing white blood cells such that the number of white blood cells per milliliter (mL) of the pure platelet solution is less than 10⁶.

In some embodiments, step (a5) involves a process of removing plasma to achieve a concentration of albumin in the pure platelet solution less than 3g/dL; or step (a5) involves a process of removing plasma to achieve a concentration of globulin in the pure platelet solution less than 2g/dL; or step (a5) involves a process of removing plasma to achieve a concentration of fibrinogen in the pure platelet solution less than 100µg/mL.

In some embodiments, step (a5) involves a process of removing plasma to achieve a concentration of albumin in the pure platelet solution less than 0.25, 0.5, 0.75, 1, 1.25, 1.5, 1.75, 2, 2.25, 2.5 or 2.75g/dL

In some embodiments, step (a5) involves a process of removing plasma to achieve a concentration of globulin in the pure platelet solution less than 0.25, 0.5, 0.75, 1, 1.25, 1.5, or 1.75g/dL.

In some embodiments, step (a5) involves a process of removing plasma to achieve a concentration of fibrinogen in the pure platelet solution less than 10, 20, 30, 40, 50, 60, 70, 80 or 90µg/mL.

In some embodiments, the solution derived from blood and containing platelets is whole blood, and the blood cell layer includes a buffy coat layer and a red blood cell layer.

In some embodiments, the activation procedure comprises: (b1') mixing collagen and thrombin with the pure platelet solution; and (b2') vortexing at room temperature for 30 minutes.

In some embodiments, the activation procedure comprises: (b1') mixing collagen and thrombin with the pure platelet solution to form a mixed solution, wherein the concentration of collagen in the mixed solution is 5-20µg/mL, and the concentration of thrombin in the mixed solution is 0.1-2U/mL; and (b2') vortexing the mixture at room temperature for 30 minutes.

In some embodiments, the activation procedure comprises: (b1') mixing collagen and thrombin with the pure platelet solution to form a mixed solution, wherein the concentration of collagen in the mixed solution is 7-12µg/mL, and the concentration of thrombin in the mixed solution is 0.5-1.5U/mL; and (b2') vortexing the mixture at room temperature for 30 minutes.

In some embodiments, the activation procedure comprises: (b1') mixing collagen and thrombin with the pure platelet solution to form a mixed solution, wherein the concentration of collagen in the mixed solution is 8, 9, 10 or 11µg/mL, and the concentration of thrombin in the mixed solution is 0.6, 0.7, 0.8, 0.9, 1, 1.1, 1.2, 1.3 or 1.4U/mL; and (b2') vortexing the mixture at room temperature for 30 minutes.

In some embodiments, the freeze-drying process comprises reducing the temperature to a value of less than or equal to -35°C and reducing the pressure to a value of less than or equal to 80mTorr.

In some embodiments, the freeze-drying process comprises reducing the temperature to a value of less than or equal to -40°C, -45°C, -50°C, -60°C, -65°C or -70°C.

In some embodiments, the freeze-drying process comprises reducing the pressure to less than or equal to 70, 60, 50, 40 or 30mTorr.

In some embodiments, the solution derived from blood and containing platelets is whole blood, apheresis platelets, leukocytes-reduced platelets apheresis, platelet-rich plasma (PRP) or any combination thereof; or the solution derived from blood and containing platelets is derived from an autologous blood sample, an allogeneic blood sample, a mammalian blood sample or a combination thereof.

In some embodiments, in step (a5), the solvent is sterile saline, water for injection, 0.45% NaCl, 4.5% hypertonic saline, sterile warm spring water or isotonic saline; or in step (a5), the platelet concentration in the pure platelet solution is 1×10⁹/mL to 10×10⁹/mL.

In some embodiments, in step (a5), the platelet concentration in the pure platelet solution is 2×10⁹/mL, 3×10⁹/mL, 4×10⁹/mL, 5×10⁹/mL, 6×10⁹/mL, 7×10⁹/mL, 8×10⁹/mL or 9×10⁹/mL.

In some embodiments, the filter membrane filtration process in step (c2) utilizes a filter membrane with a pore size of less than or equal to 0.45 µm to filter to obtain the purified platelet-derived exosome solution.

In some embodiments, the filter membrane filtration process in step (c2) utilizes a filter membrane with a pore size of less than or equal to 0.22 µm to filter to obtain the purified platelet-derived exosome solution.

The present invention futher provides a purified platelet-derived exosome dry powder prepared by a preparation method, and the preparation method includes: (a) taking one unit of a solution derived from blood and containing platelets, and performing a process of purifying platelets to obtain a pure platelet solution; wherein step (a) comprises: (a1) taking one unit of a solution derived from blood and containing platelets; (a2) performing a first sedimentation process to separate the solution derived from blood and containing platelets into a plasma layer and a blood cell layer; (a3) removing the blood cell layer to obtain a plasma layer solution; (a4) performing a second sedimentation process to cause platelets to settle and form a pellet; (a5) removing a supernatant and mixing the pellet with a solvent to obtain the pure platelet solution; (b) performing an activation procedure to activate platelets in the pure platelet solution and release exosomes to obtain an activated pure platelet solution; wherein the activation procedure is a co-treatment procedure with collagen and thrombin; (c) performing a purification process, which includes: (c1) performing a centrifugation process to remove most of platelet-associated structures, that is, removing the pellet obtained after centrifugation; and (c2) performing a filter membrane filtration process to completely remove platelet-associated structures to obtain a purified platelet-derived exosome solution.; and (d) performing a drying process on the purified platelet-derived exosome solution to obtain a purified platelet-derived exosome dry powder containing a plurality of platelet-derived exosomes.

In some embodiments, the drying process is a freeze-drying process.

In some embodiments, the activation procedure comprises: (b1') mixing collagen and thrombin with the pure platelet solution to obtain a mixed solution, wherein the concentration of collagen in the mixed solution is 5-20µg/mL (e.g., 10µg/mL), and the concentration of thrombin in the mixed solution is 0.1-2U/mL (e.g., 1U/mL); and (b2') vortexing at room temperature for 30 minutes.

In some embodiments, the activation procedure comprises: (b1') mixing collagen and thrombin with the pure platelet solution to obtain a mixed solution, wherein the concentration of collagen in the mixed solution is 6, 7, 8, 9, 10, 11, 12, 13, 14, 15, 16, 17, 18 or 19 µg/mL, and the concentration of thrombin in the mixed solution is 0.2, 0.3, 0.4, 0.5, 0.6, 0.7, 0.8, 0.9, 1, 1.1, 1.2, 1.3, 1.4, 1.5, 1.6, 1.7, 1.8 or 1.9U/mL; and (b2') vortexing at room temperature for 30 minutes.

In some embodiments, in the purified platelet-derived exosome dry powder, a particle diameter of the platelet-derived exosomes is 20-150nm, and the platelet-derived exosomes comprise an exosome marker and a platelet marker; wherein the exosome marker is at least one of CD9, CD63, and CD81 or any combination thereof, and the platelet marker is at least one of CD41 and CD42b or a combination thereof; wherein (1) per gram (g) of the purified platelet-derived exosome dry powder contains at least 1×10¹⁰ platelet-derived exosomes; or (2) per gram (g) of the purified platelet-derived exosome dry powder contains 1×10¹¹ to 1×10¹⁵ platelet-derived exosomes; or (3) per gram (g) of the purified platelet-derived exosome dry powder contains 2×10¹¹, 3×10¹¹, 4×10¹¹, 5×10¹¹, 6×10¹¹, 7×10¹¹, 8×10¹¹, 9×10¹¹, 1×10¹², 2×10¹², 3×10¹², 4×10¹², 5×10¹², 6×10¹², 7×10¹², 8×10¹², 9×10¹², 1×10¹³, 1.1×10¹³, 1.2×10¹³, 1.3×10¹³, 1.4×10¹³, 1.5×10¹³, 1.6×10¹³, 1.7×10¹³, 1.8×10¹³, 1.9×10¹³, 2×10¹³, 2.5×10¹³, 3×10¹³, 3.5×10¹³, 4×10¹³, 4.5×10¹³, 5×10¹³, 5.5×10¹³, 6×10¹³, 6.5×10¹³, 7×10¹³, 7.5×10¹³, 8×10¹³, 8.5×10¹³, 9×10¹³, 9.5×10¹³, 1×10¹⁴, 2×10¹⁴, 3×10¹⁴, 4×10¹⁴, 5×10¹⁴, 6×10¹⁴, 7×10¹⁴, 8×10¹⁴ or 9×10¹⁴ platelet-derived exosomes.

In some embodiments, (1) per gram (g) of the purified platelet-derived exosome dry powder contains at least 50µg of microRNA; or (2) the microRNA content per gram (g) of the purified platelet-derived exosome dry powder ranges from 100µg to 2,500µg.

In some embodiments, (1) the content of PDGF-BB per gram (g) of the purified platelet-derived exosome dry powder is 0.01ng to 2,000ng; or (2) the content of VEGF per gram (g) of the purified platelet-derived exosome dry powder is 50pg to 100,000pg; or (3) the content of IGF per gram (g) of the purified platelet-derived exosome dry powder is 1pg to 3,000pg; or (4) the content of TGF-β1 per gram (g) of the purified platelet-derived exosome dry powder is 50ng to 20,000ng; or (5) the content of EGF per gram (g) of the purified platelet-derived exosome dry powder is 0.1ng to 200ng.

In some embodiments, the content of PDGF-BB per gram (g) of the purified platelet-derived exosome dry powder is 0.01, 0.1, 1, 10, 20, 30, 40, 50, 60, 70, 80, 90, 1×10², 5×10², 1×10³, 1.5×10³ or 2×10³ng.

In some embodiments, the content of VEGF per gram (g) of the purified platelet-derived exosome dry powder is 50, 100, 150, 200, 250, 300, 350, 400, 450, 500, 550, 600, 650, 700, 750, 800, 850, 900, 950, 1×10³, 2×10³, 3×10³, 4×10³, 5×10³, 6×10³, 7×10³, 8×10³, 9×10³, 1×10⁴, 1.5×10⁴, 1.5×10⁴, 2×10⁴, 2.5×10⁴, 3×10⁴, 3.5×10⁴, 4×10⁴, 4.5×10⁴, 5×10⁴, 6×10⁴, 7×10⁴, 8×10⁴, 9×10⁴ or 1×10⁵pg.

In some embodiments, the content of IGF per gram (g) of the purified platelet-derived exosome dry powder is 1, 10, 20, 30, 40, 50, 60, 70, 80, 90, 1×10², 5×10², 1×10³, 1.5×10³, 2×10³, 2.5×10³ or 3×10³pg.

In some embodiments, the content of TGF-β1 per gram (g) of the purified platelet-derived exosome dry powder is 50, 100, 200, 300, 400, 500, 600, 700, 800, 900, 1×10³, 5×10³, 1×10⁴, 1.5×10⁴ or 2×10⁴ng.

In some embodiments, the content of EGF per gram (g) of the purified platelet-derived exosome dry powder is 1, 10, 20, 30, 40, 50, 60, 70, 80, 90, 100, 150 or 200ng.

In some embodiments, (1) no white blood cell-derived exosome is detected per gram of the purified platelet-derived exosome dry powder; or (2) the number of white blood cell-derived exosomes per gram of the purified platelet-derived exosome dry powder is less than or equal to 1×10¹⁴; wherein the white blood cell-derived exosome comprises a white blood cell marker; the white blood cell marker is CD45.

In some embodiments, (1) no red blood cell-derived exosome is detected per gram of the purified platelet-derived exosome dry powder; or (2) the number of red blood cell-derived exosomes per gram of the purified platelet-derived exosome dry powder is less than or equal to 1×10¹⁴; wherein the red blood cell-derived exosome comprises a red blood cell marker; the red blood cell marker is at least one of CD235ar and Annexin V or a combination thereof.

In some embodiments, (1) no exosomes other than the platelet-derived exosomes are detected per gram of the purified platelet-derived exosome dry powder; or (2) the number of exosomes other than the platelet-derived exosomes of the purified platelet-derived exosome dry powder is less than or equal to 1×10¹⁴.

The present invention further provides the use of the purified platelet-derived exosome dry powder in the manufacture of a pharmaceutical composition or health composition for administrating an effective dose to a site of an individual to alleviate inflammation or injury at the site of the individual.

In some embodiments, it is used in the manufacture of a pharmaceutical composition or health composition to relieve the degree of inflammation or injury in the respiratory tract, skin, muscles, tendons, bones, joints or ligaments of the individual.

The present invention further provides the use of the purified platelet-derived exosome dry powder in the manufacture of a pharmaceutical composition or health composition for enhancing the migration ability of dermal fibroblasts to a skin site.

In some embodiments, the skin site refers to a skin lesion site.

The present invention further provides a use of the purified platelet-derived exosome dry powder in the manufacture of a pharmaceutical composition or health composition for enhancing the expression of collagen in dermal fibroblasts.

The present invention further provides a method for preparing a purified platelet-derived exosome dry powder, which includes:
(a) taking one unit of a solution derived from blood and containing platelets, and performing a process of purifying platelets to obtain a pure platelet solution; wherein step (a) comprises:
   (a1) taking one unit of a solution derived from blood and containing platelets;
   (a2) performing a first sedimentation process to separate the solution derived from blood and containing platelets into a plasma layer and a blood cell layer;
   (a3) removing the blood cell layer to obtain a plasma layer solution;
   (a4) performing a second sedimentation process to cause platelets to settle and form a pellet;
   (a5) removing a supernatant and mixing the pellet with a solvent to obtain the pure platelet solution;
(b) performing an activation procedure to activate platelets in the pure platelet solution and release exosomes to obtain an activated pure platelet solution; wherein the activation procedure is a co-treatment procedure with collagen and adenosine diphosphate (ADP);
(c) performing a purification process to obtain a purified platelet-derived exosome solution; and
(d) performing a drying process on the purified platelet-derived exosome solution to obtain a purified platelet-derived exosome dry powder containing a plurality of platelet-derived exosomes.

In some embodiments, the activation procedure comprises:
(b1) mixing collagen and ADP with the pure platelet solution to obtain a mixed solution, wherein a concentration of collagen in the mixed solution is 5-20µg/mL, and a concentration of ADP in the mixed solution is 40-80µM; and (b2) vortexing at room temperature for 30 minutes.

In some embodiments, the activation procedure comprises:
(b1) mixing collagen and ADP with the pure platelet solution to obtain a mixed solution, wherein a concentration of collagen in the mixed solution is 7-12µg/mL, and a concentration of ADP in the mixed solution is 50-80µM; and (b2) vortexing at room temperature for 30 minutes.

In some embodiments, the activation procedure comprises:
(b1) mixing collagen and ADP with the pure platelet solution to obtain a mixed solution, wherein a concentration of collagen in the mixed solution is 8, 9, 10 or 11µg/mL, and a concentration of ADP in the mixed solution is 40, 45, 50, 55, 60, 65, 70 or 75µM; and
(b2) vortexing at room temperature for 30 minutes.

### [BRIEF DESCRIPTION OF THE DRAWING]

Fig. 1: Flow chart for preparing a purified platelet-derived exosome dry powder.
Fig. 2: The histogram of the effects of different activation methods on the concentration of platelet-derived exosomes.
Fig. 3: The histogram of the effects of the platelet-derived exosome composition obtained by co-treatment with collagen and thrombin on the migration of human dermal fibroblasts.
Fig. 4: The histogram of the compositions of exosomes in different biological solutions.
Fig. 5A: The histogram of the effects of exosomes in different biological solutions on the migration of human dermal fibroblasts.
Fig. 5B: The histogram of the effects of exosomes in different biological solutions on the proliferation of human dermal fibroblasts.
Fig. 6: The histogram of the effects of the freeze-drying process on the anti-inflammatory effect of the product.

### [DETAILED DESCRIPTION OF THE INVENTION]

The following is a detailed description of the embodiments of the present invention, as well as the technology and features of the present invention. However, the embodiments are not intended to limit the present invention, and any changes and modifications made by anyone who is familiar with the technology without departing from the spirit and scope of the present invention should be included in the scope of the present invention.

In the present invention, a purified platelet-derived exosome dry powder refers to a dry powder enriched with pure platelet-derived exosomes, for example, each gram of dry powder contains at least 1×10¹⁰ exosomes, and 80%, 85%, 90%, 95%, 96%, 97%, 98%, 99%, or 99.5% or more of them are platelet-derived exosomes.

In the present invention, the "purified platelet-derived exosome" dry powder contains fewer other exosomes than the source composition from which the dry powder is isolated, wherein "other exosomes" refer to exosomes other than platelet-derived exosomes.

In the present invention, the purified platelet-derived exosome solution refers to a solution enriched with pure platelet-derived exosomes.

In the present invention, the "purified platelet-derived exosome" solution contains fewer other exosomes than the source solution from which the solution is isolated, wherein "other exosomes" refer to exosomes other than platelet-derived exosomes.

In the present invention, "one unit" of a platelet-containing solution refers to a capacity of a container of any size for the platelet-containing solution, such as a capacity of a blood bag of the platelet-containing solution is 250 mL, and a capacity of a 50 mL centrifuge tube of the platelet-containing solution is 50 mL, and a capacity of a 1 mL container of the platelet-containing solution is 1 mL.

In the present invention, a pure platelet solution refers to a higher-purity platelet solution purified from a platelet-containing solution.

In the present invention, a platelet-associated structure refers to a platelet, a platelet fragment, or a platelet structure formed by aggregation of platelets, or any combination thereof.

In the present invention, a sedimentation process refers to a process that causes a substance to settle, such as a process that causes a substance to settle using gravity, centrifugal force, or electromagnetic force. In the present invention, "health composition" or "health product" refers to a nutritional supplement, a composition that can promote the normal physiological function in an individual's tissue after being administered to the individual, or a composition that can relieve an individual's symptoms after being administered to the individual.

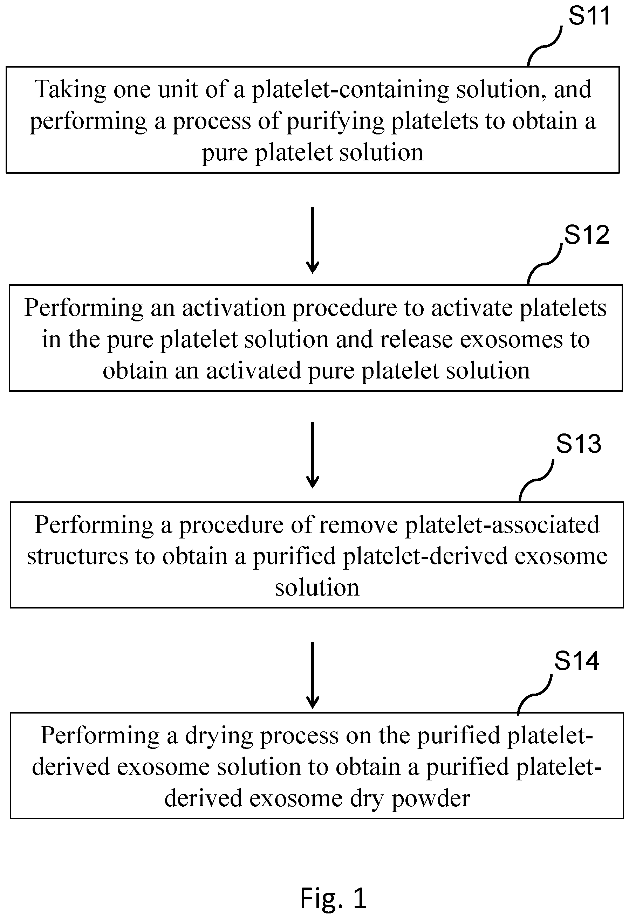

Referring to Fig. 1, a method for preparing a purified platelet-derived exosome dry powder comprises following steps:
(S11) taking one unit of a platelet-containing solution, and performing a process of purifying platelets to obtain a pure platelet solution;
(S12) performing an activation procedure to activate platelets in the pure platelet solution and release exosomes to obtain an activated pure platelet solution;
(S13) performing a procedure to remove platelet-associated structures to obtain a purified platelet-derived exosome solution; and
(S14) performing a drying process on the purified platelet-derived exosome solution to obtain a purified platelet-derived exosome dry powder containing a plurality of platelet-derived exosomes.

In some embodiments, the platelet-containing solution is derived from blood. In some embodiments, the platelet-containing solution is whole blood, apheresis platelet, leukocytes-reduced platelets apheresis, platelet-rich plasma (PRP) or any combination thereof. In some embodiments, the platelet-containing solution is derived from an autologous blood sample or an allogeneic blood sample or a combination thereof.

In some embodiments, step (S11) further comprises sub-steps:
(S111) taking one unit of a solution derived from blood and containing platelets; and
(S112) performing at least one sedimentation process to separate the solution derived from blood and containing platelets into multiple layers; wherein, the multiple layers comprise a platelet layer (e.g., the first platelet layer) and a white blood cell layer. The white blood cell layer is removed to obtain the pure platelet solution, so that the number of white blood cells in the pure platelet solution per milliliter (mL) less than 10⁶. Alternatively, performing at least one sedimentation process to separate the platelet-containing solution into multiple layers, wherein, the multiple layers include a platelet layer (e.g., the second platelet layer), and plasma and plasma protein layer. The plasma and plasma protein layer is removed to obtain the pure platelet solution, so that the concentration of albumin in the pure platelet solution is less than 3g/dL, or the concentration of globulin in the pure platelet solution is less than 2g/dL, or the concentration of fibrinogen in the pure platelet solution is less than 100µg/mL.

In some embodiments, step (S112) further comprises sub-steps:
(S1121) performing the first sedimentation process, so that the platelet-containing solution is separated into a first platelet layer (e.g., a plasma layer), a white blood cell layer (e.g., a buffy coat layer), and red blood cells layer;
(S1122) removing the buffy coat layer and the red blood cells layer to obtain a plasma layer solution;
(S1123) performing the second sedimentation process to allow the platelets to settle and form a pellet, and the pellet is the second platelet layer, and the supernatant is the plasma and plasma protein layer; and
(S1124) removing the supernatant, and mixing the pellet with a solvent to obtain the pure platelet solution.

In some embodiments, the solvent is sterile saline, water for injection, 0.45% NaCl, 4.5% hypertonic saline, sterile warm spring water, or isotonic saline, or any combination thereof. In some embodiments, a platelet concentration in the pure platelet solution is 1×10⁹/mL to 10×10⁹/mL.

In some embodiments, in step (S12), the activation procedure is a co-treatment procedure with collagen and thrombin, and step (S12) further comprises sub-steps:
(S121) mixing collagen and thrombin with the pure platelet solution to obtain a mixed solution, wherein a concentration of collagen in the mixed solution is 7-12µg/mL, and a concentration of thrombin in the mixed solution is 0.5-1.5U/mL; and
(S122) vortexing at room temperature for 30 minutes to activate platelets in the pure platelet solution and release exosomes to obtain the activated pure platelet solution.

In some embodiments, step (S13) further comprises sub-steps:
(S131) performing a centrifugation process to remove most of platelet-associated structures, that is, removing the pellet obtained after centrifugation; and
(S132) performing a filter membrane filtration process to completely remove platelet-associated structures to obtain the purified platelet-derived exosome solution.

In some embodiments, in step (S14), the drying process is a freeze-drying process. In some embodiments, the freeze-drying process comprises lowering a temperature to less than or equal to -30°C and lowering thepressure to less than or equal to 100 mTorr. In some embodiments, the freeze-dryingprocedure in step (S14) comprises lowering the temperature to less than or equal to -35°C and lowering a pressure to less than or equal to 80mTorr.

### Experiment 1: Preparation of purified platelet-derived exosome dry powder and analysis of its components

In this experiment, the following steps (S11') -(S14') were used to prepare the purified platelet-derived exosome dry powder:
(S11') performing the procedure for preparing the pure platelet solution, which comprises the following sub-steps:
   (S111') placing 250 mL of human whole blood into a blood bag containing anticoagulant;
   (S112') performing the process of purifying platelets, which comprises the following sub-steps:
      (S1121') centrifuging the human whole blood (500-1,200g, 5-8 minutes; this was the first centrifugation) using a centrifuge to separate the human whole blood into three layers; from top to bottom were a plasma layer, a buffy coat layer where white blood cells were located, and a red blood cells layer. The stratification of blood cells after centrifugation is well known to those skilled in the art, and the centrifugation conditions for the first centrifugation can be expanded to 300-1,500g for 3-10 minutes. After centrifugation, platelets were distributed in the plasma layer and adjacent to the buffy coat layer.
      (S1122') Aseptically aspirating the plasma layer to completely remove white blood cells and red blood cells, which was platelet-rich plasma (PRP) in the present disclosure, also known as the plasma layer solution in the present disclosure;
      (S1123') Detecting the total number of platelets in the plasma layer solution using an automatic hemocytometer analyzer (model: XP-300, SYSMEX). The plasma layer solution was centrifuged (1000-2,500g, 5 minutes; this was the second centrifugation) using a centrifuge to allow platelets to settle and form a pellet, that was the platelet layer, and the supernatant was the plasma and plasma protein layer. The above-mentioned centrifugation conditions for the second centrifugation can be expanded to 500-3,000g, 5-20 minutes, and the centrifugation speed must be higher than the actual centrifugation speed (300-1,500g, 3-10 minutes) used in the first centrifugation step above;
      (S1124') Completely removing the supernatant to totally take away the plasma and the protein in the plasma, and then suspending the platelets in the platelet layer with isotonic sodium chloride solution injection (to make the platelet concentration 1×10⁹/mL; 10-20 mL in total) to obtain the pure platelet solution.
(S12') Performing an activation procedure, which comprises the following sub-steps:
   (S121') mixing collagen and thrombin with the pure platelet solution to obtain a mixed solution, wherein a concentration of collagen in the mixed solution was 5-20µg/mL, and a concentration of thrombin in the mixed solution was 0.1-2U/mL, wherein a platelet concentration in the pure platelet solution was 1×10⁹/mL, and when the activation procedure was performed, the pure platelet solution contained 1×10⁹ platelets. Preferably, a concentration of collagen was 7-12µg/mL, and a concentration of thrombin was 0.5-1.5U/mL.
   (S122') vortexing (100 rpm) at room temperature for 30 minutes to activate platelets in the pure platelet solution and release exosomes to obtain the activated pure platelet solution.
(S13') performing a purification process to remove platelet-associated structures, which comprises the following sub-steps:
   (S131') centrifuging the activated pure platelet solution using a centrifuge (10,000-15,000g, 5-10 minutes) to allow the platelet-associated structures to settle and form a platelet-associated structure pellet. Centrifugation conditions can be expanded to 8,000-20,000g, 3-15 minutes.
   (S132') Carefully transferring the supernatant into the specimen bottle and filtering it with a filter membrane with a pore size of 0.45µm (PES, 25 mm, model: C0000296, Labfil^{®}) to completely remove the platelet-associated structures. The supernatant was a purified platelet-derived exosome solution. The specimen bottle contained about 1 mL of the purified platelet-derived exosome solution, and the about 1 mL of the purified platelet-derived exosome solution contained platelet-derived exosomes released by 1×10⁹ platelets. Next, a nanoparticle size analyzer (Nano Tracking Analysis, NTA, model: NanoSight NS300, Malvern) was used to perform quantitative analysis of the nanoparticles. The analysis results showed that a content of exosomes in the about 1 ml of the purified platelet-derived exosome solution was about 1×10⁹-1×10¹², and a particle size was 20-150 nm. Next, a fully automatic exosome fluorescence quantitative analyzer (ExoView) (Leprechaun) was used to analyze whether the exosomes contained biomarkers of the platelet-derived exosomes. It could be found that the exosomes had exosome markers CD9, CD63, and CD81 and platelet markers CD41 and CD42b, so the exosomes were indeed platelet-derived exosomes; moreover, the exosomes did not have a white blood cell-derived marker CD45, nor a red blood cell-derived marker CD235ar or Annexin V It can be seen that the purified platelet-derived exosome dry powder prepared in steps (S11') to (S14') of Experiment 1 of the present disclsoure did not contain white blood cell-derived exosomes, red blood cell-derived exosomes.
(S14') performing a freeze-drying process, comprising the following sub-steps:
   (S141') pre-cooling (-30 to -50°C, at least 5 hours);
   (S142') first drying (the temperature rose from the temperature gradient of step S141' to 10°C for 10-20 hours, and the vacuum value was less than or equal to 100 mTorr); and
   (S143') secondary drying (20°C, 3-5 hours, and the vacuum value was less than or equal to 100 mTorr). The dried product after freeze-drying was the purified platelet-derived exosome dry powder. A weight of the bottle of the purified platelet-derived exosome dry powder was measured using a precision electronic balance (model: ME204, Mettler). The results showed that an average weight of each bottle of the purified platelet-derived exosome dry powder was 0.0067g. After calculation, it was found that each gram of the purified platelet-derived exosome dry powder contained at least 10¹³ platelet-derived exosomes.

In this embodiment, a concentration of collagen was 10µg/mL and a concentration of thrombin was 1U/mL.

As can be seen from the above preparation process, the preparation process of the purified platelet-derived exosome dry powder comprised the steps of removing white blood cells (WBC) and plasma, activating platelets (e.g., adding collagen and thrombin), removing platelet-associated structures, and comprising the drying step (e.g., by freezing, low temperature, or vacuum drying). Therefore, the purified platelet-derived exosome dry powder contained low concentrations or even no white blood cells (WBC), plasma, and platelets.

In some embodiments, the purified platelet-derived exosome dry powder contained a low concentration or even no albumin.

In some embodiments, the purified platelet-derived exosome dry powder contained a low concentration or even no globulin.

In some embodiments, the purified platelet-derived exosome dry powder contained a low concentration or even no fibrinogen.

### Experiment 2: Effects of activation procedure on the release of platelet-derived exosomes and PDGF-BB

Step (S11') of Experiment 1-1 was performed to prepare a pure platelet solution, and a platelet concentration in the pure platelet solution was 1× 10⁹/mL. Then, the pure platelet solution was divided into five groups, namely, a control group, a calcium chloride group, an adenosine diphosphate group, a collagen group, and a thrombin group.

Control group: 1mL of the pure platelet solution was taken, and the activation procedure of step (S12') of Experiment 1-1 was not performed. That is, the pure platelet solution was not mixed with collagen and thrombin. It was only left to stand for 20-30 minutes. Then, step (S13') of Experiment 1-1 was performed to prepare a control group solution.

Calcium chloride group: 1 mL of the pure platelet solution was taken, and the activation procedure of step (S12') of Experiment 1 was not performed. That is, the pure platelet solution was not mixed with collagen and thrombin. Instead, it was mixed with calcium chloride so that a concentration of calcium chloride in the mixture reached 0.45%. Then, step (S13') of Experiment 1 was performed to prepare a calcium chloride group solution.

Adenosine diphosphate group: 1 mL of the pure platelet solution was taken, and the activation procedure of step (S12') of Experiment 1 was not performed. That is, the pure platelet solution was not mixed with collagen and thrombin. Instead, it was mixed with adenosine diphosphate so that a concentration of adenosine diphosphate in the mixture reached 60 µM. Then, step (S13') of Experiment 1 was performed to prepare an adenosine diphosphate group solution.

Collagen group: 1 mL of the pure platelet solution was taken, and the activation procedure of step (S12') of Experiment 1 was not performed. That is, the pure platelet solution was not mixed with collagen and thrombin. Instead, it is only mixed with collagen so that a collagen concentration in the mixture reached 10µg/mL. Then, step (S13') of Experiment 1 was performed to prepare a collagen group solution.

Thrombin group: 1 mL of the pure platelet solution was taken, and the activation procedure of step (S12') of Experiment 1 was not performed. That is, the pure platelet solution was not mixed with collagen and thrombin. Instead, it is only mixed with thrombin so that a thrombin concentration in the mixed solution reached 1U/mL. Then, step (S13') of Experiment 1 was performed to prepare a thrombin group solution.

A concentration of platelet-derived exosomes in each solution was analyzed using a nanoparticle size analyzer (tunable resistive pulse sensing, TRPS, model: Exoid, IZON Science), and a concentration of a growth factor (PDGF-BB) in each solution was analyzed using enzyme-linked immunosorbent assay (ELISA).

The experimental results showed that a change trend of platelet-derived exosomes and a change trend of PDGF-BB release fold were independent of each other in solutions prepared by different activation methods. Therefore, the inventor further conducted following experiments to find out the best method for preparing platelet-derived exosomes.

### Experiment 3: Effects of different activation methods on exosome concentration.

Step (S11') of Experiment 1-1 was performed to prepare a pure platelet solution, and a platelet concentration in the pure platelet solution was 1×10⁹/mL. Next, the pure platelet solution was divided into a control group and 7 experimental groups, namely a calcium chloride group, an adenosine diphosphate group, a collagen group, a thrombin group, a calcium chloride + adenosine diphosphate group, an adenosine diphosphate + collagen group, and a collagen + thrombin group.

Control group: 1mL of the pure platelet solution was taken, and the activation procedure of step (S12') of Experiment 1-1 was not performed. Instead, it was only left to stand for 20-30 minutes. Next, step (S13') of Experiment 1-1 was performed to prepare a control group solution.

Calcium chloride (CaCl₂) group: 1 mL of the pure platelet solution was taken, and the activation procedure of step (S12') of Experiment 1 was not performed. That is, the pure platelet solution was not mixed with collagen and thrombin. Instead, it was mixed with calcium chloride so that a concentration of calcium chloride in the mixture reached 0.45%, and placed on an orbital shaker (model: DSR-D-N1, brand DIGISYSTEM) at room temperature with a speed of 100 rpm for 20 to 30 minutes. Then, step (S13') of Experiment 1 was carried out to prepare a calcium chloride group solution.

Adenosine diphosphate (ADP) group: 1 mL of the pure platelet solution was taken, and the activation procedure of step (S12') of Experiment 1 was not performed. That is, the pure platelet solution was not mixed with collagen and thrombin. Instead, it was mixed with adenosine diphosphate so that a concentration of adenosine diphosphate (ADP) in the mixture reached 60 µM, and placed on an orbital shaker (model: DSR-D-N1, DIGISYSTEM) at room temperature with a speed of 100 rpm for 20 to 30 minutes. Then, step (S13') of Experiment 1 was performed to prepare an adenosine diphosphate (ADP) group solution.

Collagen group: 1 mL of the pure platelet solution was taken, and the activation procedure of step (S12') of Experiment 1 was not performed. That is, the pure platelet solution was not mixed with collagen and thrombin. Instead, it was only mixed with collagen so that a collagen concentration in the mixture reached 10µg/mL, and placed on an orbital shaker (model: DSR-D-N1, DIGISYSTEM) at room temperature with a speed of 100 rpm for 20 to 30 minutes. Then, step (S13') of Experiment 1 was performed to prepare a collagen group solution.

Thrombin group: 1 mL of the pure platelet solution was taken, and the activation procedure of step (S12') of Experiment 1 was not performed. That is, the pure platelet solution was not mixed with collagen and thrombin. Instead, it was only mixed with thrombin so that a thrombin concentration in the mixed solution reached 1U/mL, and placed on an orbital shaker (model: DSR-D-N1, DIGISYSTEM) at room temperature with a speed of 100 rpm for 20 to 30 minutes. Then, step (S13') of Experiment 1 was performed to prepare a thrombin group solution.

Calcium chloride + adenosine diphosphate group: 1 mL of the pure platelet solution was taken, and the activation procedure of step (S12') of Experiment 1 was not performed. That is, the pure platelet solution was not mixed with collagen and thrombin. Instead, it was mixed with calcium chloride and adenosine diphosphate so that a concentration of calcium chloride in the mixture reached 0.45% and a concentration of adenosine diphosphate in the mixture reached 60 µM, and placed on an orbital shaker at room temperature with a speed of 100 rpm for 20 to 30 minutes. Then, step (S13') of Experiment 1 was performed to prepare a calcium chloride + adenosine diphosphate group solution.

Adenosine diphosphate + collagen group: 1 mL of the pure platelet solution was taken, and the activation procedure of step (S12') of Experiment 1 was not performed. That is, the pure platelet solution was not mixed with collagen and thrombin. Instead, it was mixed with adenosine diphosphate and collagen to make a concentration of adenosine diphosphate in the mixture reached 60 µM and a concentration of collagen in the mixture reached 10 µg/mL, and placed on an orbital shaker at room temperature with a speed of 100 rpm for 20 to 30 minutes. Then, step (S13') of Experiment 1 was performed to prepare an adenosine diphosphate + collagen group solution.

Collagen + thrombin group: 1 mL of the pure platelet solution was taken and steps (S12') to (S13') of Experiment 1 were performed to prepare a collagen + thrombin group solution (i.e., the purified platelet-derived exosome solution of the present disclosure).

Nanoparticle quantitative analysis was performed using a nanoparticle size analyzer (tunable resistive pulse sensing, TRPS, model: Exoid, IZON Science) to analyze a concentration of platelet-derived exosomes in each group solution.

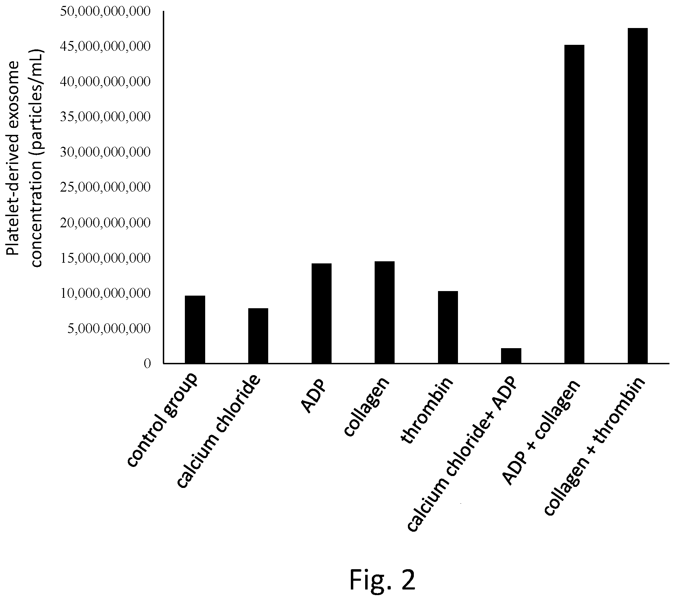

The experimental results are shown in Table 1 and Fig. 2.

**Table 1**

| Group | Concentration of platelet-derived exosomes (particles/mL) |
|---|---|
| Control group | 9,610,000,000 |
| Calcium chloride group | 7,850,000,000 |
| Adenosine diphosphate group | 14,200,000,000 |
| Collagen group | 14,500,000,000 |
| Thrombin group | 10,300,000,000 |
| Calcium chloride + adenosine diphosphate group | 2,180,000,000 |
| Adenosine diphosphate + collagen group | 45,200,000,000 |
| Collagen + thrombin group | 47,600,000,000 |

Please refer to Fig. 2. Fig. 2 is the histogram of the effects of different activation methods on the concentration of platelet-derived exosomes.

The results in Table 1 and Fig. 2 show that compared with the control group, the activation methods such as calcium chloride, adenosine diphosphate, collagen, thrombin, and calcium chloride + adenosine diphosphate were unable to significantly increase the concentration of the platelet-derived exosomes. Unexpectedly, by adding adenosine diphosphate and collagen, or collagen and thrombin, the release of platelet-derived exosomes was as high as 21 or 22 times (calculation method: 45,200,000,000÷2,180,000,000=20.7 or 47,600,000,000÷2,180,000,000=21.8). That is, the activation procedure of adding adenosine diphosphate and collagen, or collagen and thrombin could significantly increase the release of platelet-derived exosome, which had unexpected effects.

### Experiment 4: Effects of platelet-derived exosome composition obtained by co-treatment with collagen and thrombin on relieving inflammatory response and tissue repair

### Experiment 4-1: Effects of platelet-derived exosome composition obtained by co-treatment with collagen and thrombin on relieving inflammation

Human dermal fibroblast (HDF) (purchased from ScienCell Research Laboratories, Inc., product number 2320) was cultured in Dulbecco's Modified Eagle Medium (DMEM) (Corning 10-013CV) containing 2% fetal bovine serum (FBS), and then replaced with fresh medium for later use. The above-mentioned human dermal fibroblast (HDF) was an adherent cell.

Step (S11') of Experiment 1 was performed to prepare a pure platelet solution, and a platelet concentration in the pure platelet solution was 1×10⁹/mL. Then, the pure platelet solution was divided into (1) a control group and (2) a collagen + thrombin group.

Control group: 1 mL of the pure platelet solution was taken, and the activation procedure of step (S12') of Experiment 1 was not performed. Instead, it was only left to stand for 20-30 minutes. Then, step (S13') of Experiment 1 was performed to prepare a control group solution. The control group solution was mixed with DMEM culture medium (containing 2% FBS) (a volume ratio of the control group solution to the DMEM culture medium was 1:19) to prepare a control group's platelet-derived exosome medium.

Collagen + thrombin group: 1 mL of the pure platelet solution was taken, and the activation procedure of steps (S12')-(S13') of Experiment 1 was performed to prepare a collagen + thrombin group solution (i.e., the purified purified platelet-derived exosome solution of the present disclsoure). The collagen + thrombin group solution was mixed with DMEM culture medium (containing 2% FBS) (a volume ratio of the collagen + thrombin group solution to the DMEM culture medium was 1: 19) to prepare a collagen and thrombin group's platelet-derived exosome medium.

A 96-well plate was divided into untreated wells, LPS control wells, control group's platelet-derived exosome solution wells, and collagen + thrombin group's platelet-derived exosome solution wells, with 2 replicates each. Human dermal fibroblasts (HDF) were added to the untreated wells, LPS control wells, control group's platelet-derived exosome solution wells, and collagen + thrombin group's platelet-derived exosome solution wells, respectively, so that each well contained the same number of cells. The cells were cultured in an incubator at 37°C with 5% carbon dioxide (CO₂) for 24 hours.

The used medium was removed. The cells in the LPS control wells, the control group's platelet-derived exosome solution wells, and the collagen + thrombin group's platelet-derived exosome solution wells were suspended with DMEM culture medium (containing 2% FBS) containing 5µg/mL LPS, and the cells in the untreated wells were suspended with DMEM culture medium (containing 2% FBS) without LPS. The cells were cultured in an incubator at 37°C with 5% carbon dioxide (CO₂) for 1 hour. Then, the following steps are performed on each group.

Untreated wells: the used medium was removed. The cells in the untreated wells (control group wells) were suspended with DMEM culture medium (containing 2% FBS) and cultured in an incubator at 37°C with 5% carbon dioxide (CO₂) for 24 hours.

LPS control wells: the used medium was removed. The cells in the LPS control wells were suspended with DMEM culture medium (containing 2% FBS) and cultured in an incubator at 37°C with 5% carbon dioxide (CO₂) for 24 hours.

Control group's platelet-derived exosome solution wells: the used medium was removed. The cells in the control group's platelet-derived exosome solution wells were suspended with the control group's exosome medium and cultured in an incubator at 37°C with 5% carbon dioxide (CO₂) for 24 hours.

Collagen + thrombin group's platelet-derived exosome solution wells: the used medium was removed. The cells in the collagen + thrombin group's platelet-derived exosome solution wells were suspended with the collagen + thrombin group's exosome medium and cultured in an incubator at 37°C with 5% carbon dioxide (CO₂) for 24 hours.

The cell culture mediums in the untreated wells, LPS control wells, control group's platelet-derived exosome solution well, and collagen + thrombin group's platelet-derived exosome solution well were transferred into microcentrifuge tubes, respectively. Human interleukin-6 (IL-6, an inflammatory factor) ELISA Kit (product number ab178013, Abeam) was used to detect the concentration of human IL-6 in the cell culture mediums. A multimode microplate reader (model: Varioskan LUX, Thermo Scientific) was then used for analysis, and an absorbance value was set at 450 nm. A regression curve between absorbance and a concentration of inflammatory factor IL-6 was established according to the product manual of Human IL-6 ELISA kit, and then the absorbance was converted into the concentration of inflammatory factor IL-6.

The data showed that the collagen + thrombin group's platelet-derived exosome solution could inhibit the inflammatory response of human fibroblasts, thereby alleviating the inflammation of skin tissue. The inventor expects that after inducing an inflammatory response in fibroblasts from other human tissues with stimulants (e.g., LPS), the collagen + thrombin group's platelet-derived exosome solution can also inhibit the induced inflammatory response, thereby alleviating inflammation in the other tissues.

### Experiment 4-2: Effects of platelet-derived exosome composition obtained by co-treatment with collagen and thrombin on cell migration

Cell migration and proliferation are beneficial to tissue repair (please refer to Reference 1). This experiment used human dermal fibroblasts (HDF) the same as that in Experiment 4-1. Human dermal fibroblasts were cultured in Dulbecco's Modified Eagle medium (DMEM, Corning 10-013CV) containing 2% fetal bovine serum (FBS), and then replaced with fresh medium for later use.

A 96-well plate containing a cell culture insert was divided into untreated wells, control group's platelet-derived exosome solution wells, and collagen + thrombin group's platelet-derived exosome solution wells, with 3 replicates each. Human dermal fibroblasts (HDF) were added to the untreated wells, the control group's platelet-derived exosome solution wells, and the collagen + thrombin group's platelet-derived exosome solution wells, so that each well contained the same number of cells, and the cells were cultured in an incubator at 37°C with 5% carbon dioxide (CO₂) for 24 hours. The used medium and the cell culture insert of each group were removed, and the plate was photographed by using a microscope. It was confirmed that in each group of wells, the area originally occupied by the cell culture insert was not covered by cells. Then, the following steps were performed on each group.

Untreated wells: the cells were cultured with DMEM culture medium (containing 2% FBS) in an incubator at 37°C with 5% carbon dioxide (CO₂) for 6 hours.

Control group's platelet-derived exosome solution wells: the cells in the control group's platelet-derived exosome solution wells were suspended using the control group's platelet-derived exosome medium described in Experiment 4-1. The cells were cultured in an incubator at 37°C with 5% carbon dioxide (CO₂) for 6 hours.

Collagen + thrombin group's platelet-derived exosome solution wells: the cells in the collagen + thrombin group's platelet-derived exosome solution wells were suspended using the collagen and thrombin group's platelet-derived exosome medium described in Experiment 4-1. The cells were cultured in an incubator at 37°C with 5% carbon dioxide (CO₂) for 6 hours.

Those were photographed using a microscope, and a cell migration coverage area of each group was calculated. The data were presented as mean ± SD and statistically analyzed by t test. The group marked with "**" indicated a statistical difference (p < 0.01) compared with the control group's platelet-derived exosome solution wells.

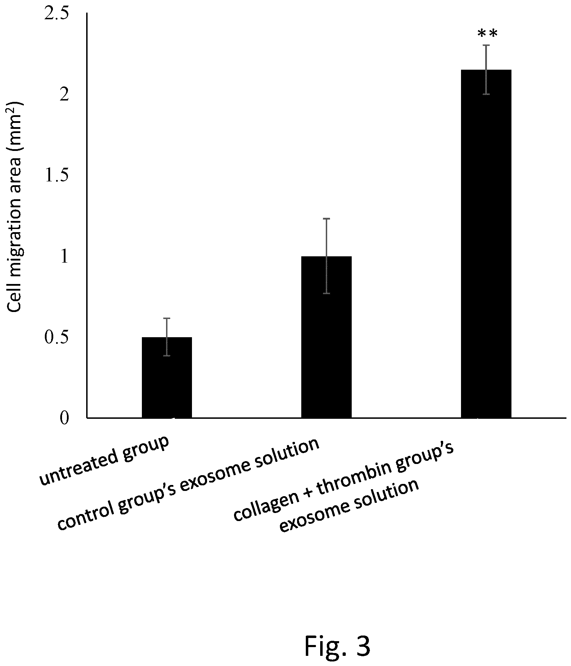

The experimental results are shown in Fig. 3. Fig. 3 is the histogram of the effects of the platelet-derived exosome composition obtained by co-treatment with collagen and thrombin on the migration of human dermal fibroblasts.

The results in Fig. 3 showed that the average cell migration area of the untreated group was 0.5 mm²; the average cell migration area of the control group's platelet-derived exosome solution group was 1.00 mm²; the average cell migration area of the collagen + thrombin group's platelet-derived exosome solution group was 2.15 mm², and there was a significant difference (p < 0.01) in the cell migration area between the collagen + thrombin group's platelet-derived exosome solution group and the control group's platelet-derived exosome solution group. It can be seen that the platelet-derived exosome composition obtained by the activation procedure of co-treatment with collagen and thrombin in the present disclosure could promote the migration reaction of human dermal fibroblasts, thereby promoting tissue repair. Moreover, from Experiment 2, it can be seen that the cell migration reaction presented in Fig. 3 was triggered by platelet-derived exosomes, not by PDGF-BB.

### Experiment 4-3: Effects of platelet-derived exosome composition obtained by co-treatment with collagen and thrombin on cell proliferation

The experiment was conducted using human dermal fibroblasts (HDF) the same as that in Experiment 4-1. Human dermal fibroblasts were cultured in Dulbecco's Modified Eagle medium (DMEM, Corning 10-013CV) containing 2% fetal bovine serum (FBS), and then replaced with fresh medium for later use.

A 96-well plate was divided into untreated wells, control group's platelet-derived exosome solution wells, and collagen + thrombin group's platelet-derived exosome solution wells, with 2 replicates each. Human dermal fibroblasts (HDF) were added to the untreated wells, the control group's platelet-derived exosome solution wells, and the collagen + thrombin group's platelet-derived exosome solution wells, so that each well contained the same number of cells, and the cells were cultured in an incubator at 37°C with 5% carbon dioxide (CO₂) for 24 hours. Then, the following steps were performed on each group.

Untreated wells: the used medium was removed. The cells were cultured with DMEM culture medium (containing 2% FBS) in an incubator at 37°C with 5% carbon dioxide (CO₂) for 6 hours.

Control group's platelet-derived exosome solution wells: the used medium was removed. The cells in the control group's platelet-derived exosome solution wells were suspended with the control group's platelet-derived exosome medium described in Experiment 4-1. The cells were cultured in an incubator at 37°C with 5% carbon dioxide (CO₂) for 6 hours.

Collagen + thrombin group's platelet-derived exosome solution wells: The cells in the collagen + thrombin group's platelet-derived exosome solution wells were suspended with the collagen and thrombin group's platelet-derived exosome medium described in Experiment 4-1. The cells were cultured in an incubator at 37°C with 5% carbon dioxide (CO₂) for 6 hours.

The used medium was removed. 90µL DMEM culture medium and 10µL CCK-8 buffer (DOJINDO, product number CK04) were added to each well. The cells were cultured in an incubator at 37°C with 5% carbon dioxide (CO₂) for 4 hours. A multimode microplate reader (model: Varioskan LUX, Thermo Scientific) was used for analysis, and an absorbance value was set at 450 nm. A regression curve between absorbance and a cell number was established according to the product manual of CCK-8 buffer, and then the absorbance was converted into the number of viable cells.

The experimental results showed that the platelet-derived exosome composition obtained by co-treatment with collagen and thrombin in the present disclosure could promote the proliferation of human dermal fibroblasts and thus promote tissue repair.

### Experiment 5: Effects of exosomes in different biological solutions on alleviating inflammation, and on migration and proliferation of human dermal fibroblasts

### Experiment 5-1: Analysis of exosome composition in different biological solutions

This experiment was divided into two groups, namely a pure platelet group and a platelet plasma group. The following steps were performed on each group.

Pure platelet group: 1 mL of the pure platelet solution described in Experiment 1 was taken, and steps (S12')-(S13') of Experiment 1 were performed to prepare a pure platelet group's exosome solution (i.e., the purified platelet-derived exosome solution of the present disclosure).

Platelet plasma group: 1 mL of platelet-rich plasma (PRP) described in Experiment 1 was taken. Then, steps (S12')-(S13') of Experiment 1 were directly performed to prepare a platelet plasma group's exosome solution.

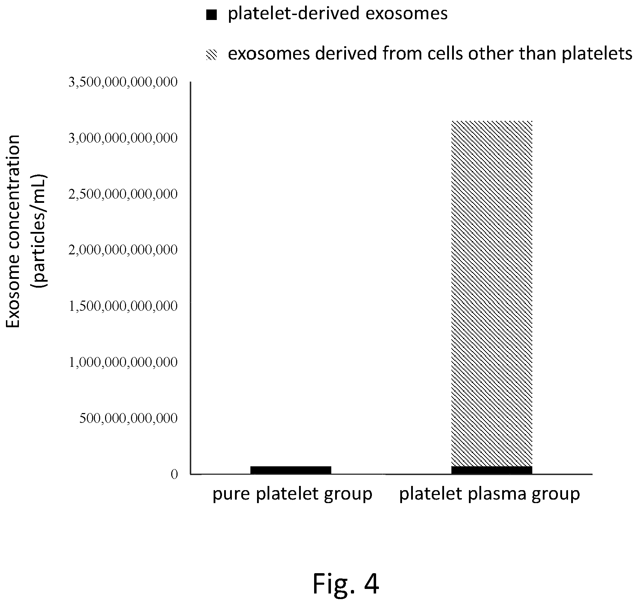

The exosome concentration in each group's solution was analyzed using a nanoparticle size analyzer (tunable resistive pulse sensing, TRPS, model: Exoid, IZON Science). Then, exosome biomarkers in the pure platelet group's exosome solution or the platelet plasma group's exosome solution were analyzed using a fully automatic exosome fluorescence quantitative analyzer (ExoView) (Leprechaun). The experimental results showed that the exosomes in the pure platelet group's exosome solution contained exosome markers CD9, CD63, and/or CD81 and platelet markers CD41 and CD42b, but did not contain a white blood cell-derived marker CD45 and at least one of red blood cell-derived exosome markers CD235ar and Annexin V It indicated that the exosomes in the pure platelet group were 100% platelet-derived. On the other hand, the exosomes in the platelet plasma group's exosome solution contained at least one of red blood cell-derived markers CD235ar and Annexin V, or a white blood cell-derived marker CD45. The experimental results are shown in Table 2 and Fig. 4. Fig. 4 is the histogram of the compositions of exosomes in different biological solutions. The experimental results showed that only 2.23% of the exosomes in the platelet plasma group were platelet-derived, while 97.77% were exosomes derived from other cell types.

**Table 2**

| Group | Concentration of exosomes derived from platelets (particles/mL) | Concentration of exosomes derived from cells other than platelets (particles/mL) |
|---|---|---|
| Pure platelet group | 70,100,000,000 | 0 |
| Platelet plasma group | 70,100,000,000 | 3,079,900,000,000 |

### Experiment 5-2: Effects of exosomes in different biological solutions on alleviating inflammation in human dermal fibroblasts

The concentration of the pure platelet group's exosome solution prepared in Experiment 5-1 and the concentration of the platelet plasma group's exosome solution prepared in Experiment 5-1 were adjusted using isotonic sodium chloride solution injection so that the exosome concentrations in both solutions were the same (the total number of exosomes per unit volume was the same).

The experiment was conducted using human dermal fibroblasts (HDF) the same as that in Experiment 4-1. Human dermal fibroblasts were cultured in Dulbecco's Modified Eagle medium (DMEM, Corning 10-013CV) containing 2% fetal bovine serum (FBS), and then replaced with fresh medium for later use.

A 96-well plate was divided into untreated wells, LPS control wells, platelet plasma experimental wells and purified platelet experimental wells, with 2 replicates each. Human dermal fibroblasts (HDF) were added to the untreated wells, LPS control wells, platelet plasma experimental wells, and purified platelet experimental wells, so that each well contained the same number of cells. The cells were cultured in an incubator at 37°C with 5% carbon dioxide (CO₂) for 24 hours.

The used medium was removed. The cells in the LPS control wells, platelet plasma experimental wells, and purified platelet experimental wells were suspended with DMEM culture medium (containing 2% FBS) containing 5µg/mL LPS, and the cells in the untreated wells were suspended with DMEM culture medium without LPS (containing 2%). The cells were cultured in an incubator at 37°C with 5% carbon dioxide (CO₂) for 24 hours. Then, the following steps are performed on each group.

Untreated wells: the used medium was removed. The cells in the untreated wells were suspended with DMEM culture medium (containing 2% FBS) and cultured in an incubator at 37°C with 5% carbon dioxide (CO₂) for 24 hours.

LPS control wells: the used medium was removed. The cells in the LPS control wells were suspended with DMEM culture medium (containing 2% FBS) and cultured in an incubator at 37°C with 5% carbon dioxide (CO₂) for 24 hours.

Platelet plasma experimental wells: the platelet plasma group's exosome solution was mixed with DMEM culture medium (containing 2% FBS) (a volume ratio of the platelet plasma group's exosome solution to DMEM culture medium was 1:19) to prepare a platelet plasma group medium. The used medium was removed. The cells in the platelet plasma experimental wells were suspended with the platelet plasma group medium and cultured in an incubator at 37°C with 5% carbon dioxide (CO₂) for 24 hours.

Purified platelet experimental wells: The pure platelet group's exosome solution was mixed with DMEM culture medium (containing 2% FBS) (a volume ratio of the pure platelet group's exosome solution to DMEM culture medium was 1: 19) to prepare purified platelet experimental medium. The used medium was removed. The cells in the purified platelet experimental wells were suspended with the purified platelet experimental medium and cultured in an incubator at 37°C with 5% carbon dioxide (CO₂) for 24 hours.

The cell culture mediums in the untreated wells (control wells), LPS control wells, platelet plasma experimental wells and purified platelet experimental wells were transferred into microcentrifuge tubes, respectively. Human interleukin-6 (IL-6, an inflammatory factor) ELISA Kit (product number ab178013, Abeam) was used to detect a concentration of human IL-6 in the cell culture mediums. A multimode microplate reader (model: Varioskan LUX, Thermo Scientific) was then used for analysis, and an absorbance value was set at 450 nm. A regression curve between absorbance and a concentration of inflammatory factor IL-6 was established according to the product manual of Human IL-6 ELISA kit, and then the absorbance was converted into the concentration of inflammatory factor IL-6.

The experimental results showed that the pure platelet-derived exosome composition (i.e., exosomes from cells other than platelets were removed) could effectively alleviate inflammatory responses.

### Experiment 5-3: Effects of exosomes in different biological solutions on migration of human dermal fibroblasts

The concentration of the pure platelet group's exosome solution prepared in Experiment 5-1 and the concentration of the platelet plasma group's exosome solution prepared in Experiment 5-1 were adjusted using isotonic sodium chloride solution injection so that the exosome concentrations in both solutions were the same (the total number of exosomes per unit volume was the same).

The experiment was conducted using human dermal fibroblasts (HDF) the same as that in Experiment 4-1. Human dermal fibroblasts were cultured in Dulbecco's Modified Eagle medium (DMEM, Corning 10-013CV) containing 2% fetal bovine serum (FBS), and then replaced with fresh medium for later use.

A 96-well plate containing a cell culture insert was divided into untreated wells, platelet plasma experimental wells and purified platelet experimental wells, with 3 replicates each. Human dermal fibroblasts (HDF) were added to the untreated wells, platelet plasma experimental wells, and purified platelet experimental wells, so that each well contained the same number of cells. The cells were cultured in an incubator at 37°C with 5% carbon dioxide (CO₂) for 24 hours. The used medium and the cell culture insert of each group were removed, and the plate was photographed by using a microscope. It was confirmed that in each group of wells, the area originally occupied by the cell culture insert was not covered by cells. Then, the following steps were performed on each group.

Untreated wells: the cells were cultured with DMEM culture medium (containing 2% FBS) in an incubator at 37°C with 5% carbon dioxide (CO₂) for 6 hours.

Platelet plasma experimental wells: the platelet plasma group's exosome solution was mixed with DMEM culture medium (containing 2% FBS) (a volume ratio of the platelet plasma group's exosome solution to DMEM culture medium was 1:19) to prepare platelet plasma experimental medium. The used medium was removed. The cells in the platelet plasma experimental wells were suspended with the platelet plasma experimental medium and cultured in an incubator at 37°C with 5% carbon dioxide (CO₂) for 6 hours.

Purified platelet experimental wells: The pure platelet group's exosome solution was mixed with DMEM culture medium (containing 2% FBS) (a volume ratio of the pure platelet group's exosome solution to DMEM culture medium was 1: 19) to prepare purified platelet experimental medium. The used medium was removed. The cells in the purified platelet experimental wells were suspended with the purified platelet experimental medium and cultured in an incubator at 37°C with 5% carbon dioxide (CO₂) for 6 hours.

Those were photographed using a microscope, and a cell migration coverage area of each group was calculated. The data were presented as mean ± SD and statistically analyzed by t test. The group marked with "*" indicated a statistical difference (p < 0.05) compared with the untreated group.

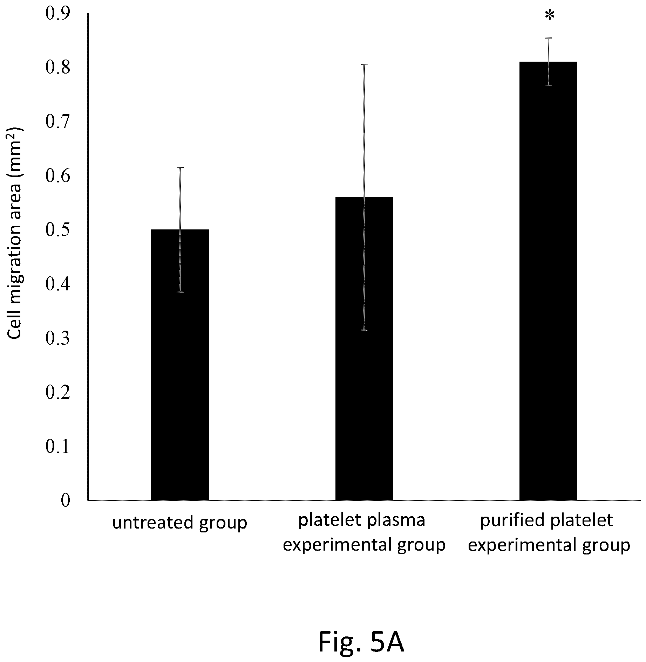

The experimental results are shown in Fig. 5A. Fig. 5A is the histogram of the effects of exosomes in different biological solutions on the migration of human dermal fibroblasts.

As shown in Fig. 5A, the average cell migration area of the untreated group was 0.5 mm²; the average cell migration area of the platelet plasma experimental group was 0.56 mm²; and the average cell migration area of the purified platelet experimental group was 0.81 mm², and there was a significant difference (p<0.05) in the cell migration area between the purified platelet experimental group and the untreated group. It can be seen that the pure platelet-derived exosome composition (i.e., exosomes from cells other than platelets were removed) could promote the migration response of human dermal fibroblasts and further promote tissue repair, which had unexpected effects.

### Experiment 5-4: Effects of exosomes in different biological solutions on proliferation of human dermal fibroblasts

The concentration of the pure platelet group's exosome solution prepared in Experiment 5-1 and the concentration of the platelet plasma group's exosome solution prepared in Experiment 5-1 were adjusted using isotonic sodium chloride solution injection so that the exosome concentrations in both solutions were the same (the total number of exosomes per unit volume was the same).

The experiment was conducted using human dermal fibroblasts (HDF) the same as that in Experiment 4-1. Human dermal fibroblasts were cultured in Dulbecco's Modified Eagle medium (DMEM, Corning 10-013CV) containing 2% fetal bovine serum (FBS), and then replaced with fresh medium for later use.

A 96-well plate was divided into untreated wells, platelet plasma experimental wells, and purified platelet experimental wells, with 2 replicates each. Human dermal fibroblasts (HDF) were added to the untreated wells, platelet plasma experimental wells, and purified platelet experimental wells, so that each well contained the same number of cells. The cells were cultured in an incubator at 37°C with 5% carbon dioxide (CO₂) for 24 hours. Then, the following steps were performed on each group.

Untreated wells: the used medium was removed. The cells were cultured in DMEM culture medium (containing 2% FBS) at 37°C in an incubator with 5% carbon dioxide (CO₂) for 6 hours.

Platelet plasma experimental wells: the used medium was removed. The cells in the platelet plasma experimental wells were suspended with the platelet plasma experimental medium described in Experiment 5-3 and cultured in an incubator at 37°C with 5% carbon dioxide (CO₂) for 6 hours.

Purified platelet experimental wells: the used medium was removed. The cells in the purified platelet experimental wells were suspended with the purified platelet experimental medium described in Experiment 5-3 and cultured in an incubator at 37°C with 5% carbon dioxide (CO₂) for 6 hours.

The used medium was removed. 90µL DMEM culture medium and 10µL CCK-8 buffer (DOJINDO, product number CK04) were added to each well. The cells were cultured in an incubator at 37°C with 5% carbon dioxide (CO₂) for 4 hours. A multimode microplate reader (model: Varioskan LUX, Thermo Scientific) was used for analysis, and an absorbance value was set at 450 nm. A regression curve between absorbance and a cell number was established according to the product manual of CCK-8 buffer, and then the absorbance was converted into the number of viable cells.

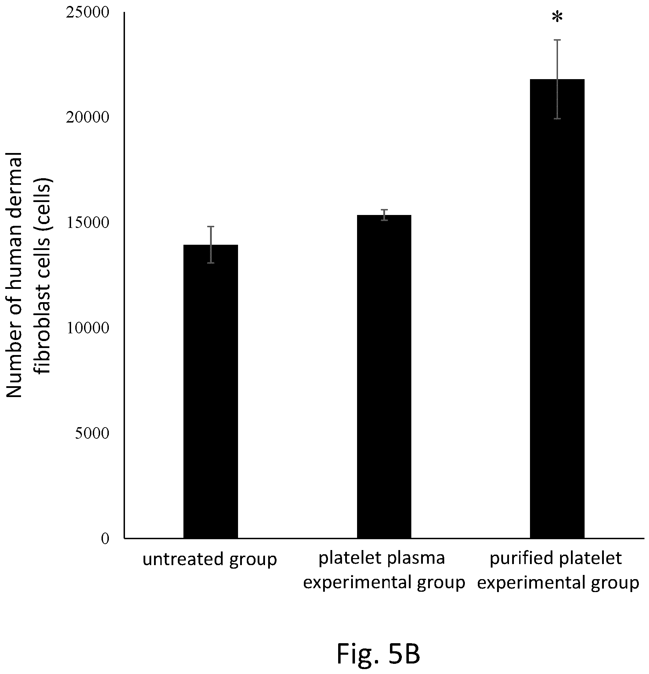

The experimental results are shown in Fig.5B. Fig. 5B is the histogram of the effects of exosomes in different biological solutions on the proliferation of human dermal fibroblasts.

As shown in Fig. 5B, the average number of cells in the untreated group was 13,947; the average number of cells in the platelet plasma experimental group was 15,357; and the average number of cells in the purified platelet experimental group was 21,797. There was a significant difference (p<0.05) in the cell number between the purified platelet experimental group and the untreated group. It can be seen that the pure platelet-derived exosome composition (i.e., exosomes from cells other than platelets were removed) could promote proliferation response of human dermal fibroblasts and further promote tissue repair, which had unexpected effects.

### Experiment 6: Effects of freeze-drying process on the anti-inflammatory effect of the product

A purified platelet-derived exosome solution was prepared according to steps (S11') to (S13') of Experiment 1.

Human dermal fibroblasts required for this experiment were prepared according to the experimental procedure of Experiment 4-1. A 96-well plate was divided into untreated wells, LPS control wells, non-freeze-drying wells, and freeze-drying wells, with 2 replicates each. Human dermal fibroblasts (HDF) were added to the untreated wells, LPS control wells, non-freeze-drying wells, and freeze-drying wells, respectively, so that each well contained the same number of cells. The cells were cultured in an incubator at 37°C with 5% carbon dioxide (CO₂) for 24 hours.

The used medium was removed. The cells in the LPS control wells, non-freeze-drying wells, and freeze-drying wells were suspended with DMEM culture medium (containing 2% FBS) containing 5µg/mL LPS, and the cells in the untreated wells were suspended with DMEM culture medium (containing 2% FBS) without LPS. The cells were cultured in an incubator at 37°C with 5% carbon dioxide (CO₂) for 1 hour. Then, the following steps are performed on each group.

Untreated wells: The used medium was removed. DMEM culture medium (containing 2% FBS) was added to the untreated wells, and the cells were cultured in an incubator at 37°C with 5% carbon dioxide (CO₂) for 24 hours.

LPS control wells: The used medium was removed. DMEM culture medium (containing 2% FBS) was added to the LPS control wells, and the cells were cultured in an incubator at 37°C with 5% carbon dioxide (CO₂) for 24 hours.

Non-freeze-drying wells: The used medium was removed. 1 mL of the purified platelet-derived exosome solution was taken and mixed with DMEM culture medium (containing 2% FBS) (a volume ratio of purified platelet-derived exosome solution to DMEM culture medium was 1:19) to obtain purified platelet-derived exosome medium, and the purified platelet-derived exosome medium was added to the non-freeze-drying wells, and the cells were cultured in an incubator at 37°C with 5% carbon dioxide (CO₂) for 24 hours.

Freeze-drying wells: The used medium was removed. 1 mL of the purified platelet-derived exosome solution was taken and step (S14') of Experiment 1 was performed to prepare a purified platelet-derived exosome dry powder. The purified platelet-derived exosome dry powder was re-dissolved with normal saline injection to prepare 1 mL of a purified platelet-derived exosome dry powder solution. 1 mL of the purified platelet-derived exosome dry powder solution was mixed with DMEM culture medium (containing 2% FBS) (a volume ratio of the purified platelet-derived exosome dry powder solution to DMEM culture medium was 1:19) to obtain purified platelet-derived exosome dry powder medium, and the purified platelet-derived exosome dry powder medium was added to freeze-drying wells, and the cells were cultured at 37°C with 5% carbon dioxide (CO₂) for 24 hours.

The cell culture mediums in the untreated wells, LPS control wells, non-freeze-drying wells, and freeze-drying wells were transferred into microcentrifuge tubes, respectively. Human interleukin-6 (IL-6, an inflammatory factor) ELISA Kit (product number ab178013, Abeam) was used to detect a concentration of human IL-6 in the cell culture mediums. A multimode microplate reader (model: Varioskan LUX, Thermo Scientific) was used for analysis, and an absorbance value was set at 450 nm. A regression curve between absorbance and a concentration of inflammatory factor IL-6 was established according to the product manual of Human IL-6 ELISA kit, and then the absorbance was converted into the concentration of inflammatory factor IL-6.

The data were presented as mean ± SD and statistically analyzed by t test. The group marked with "**" indicated a statistical difference (p < 0.01) compared with the non-freeze-drying group.

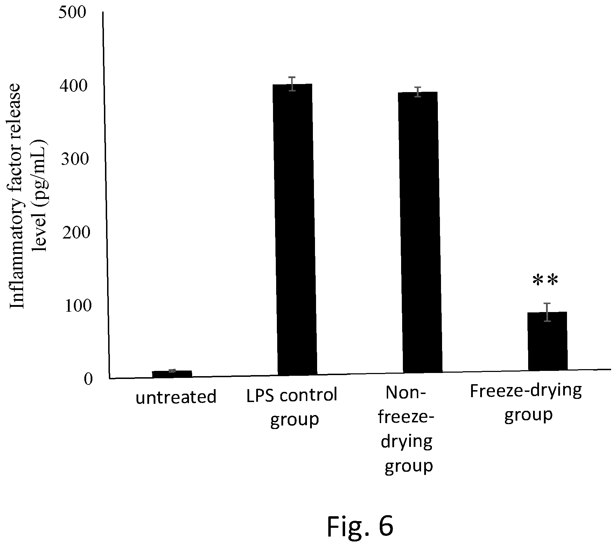

For the experimental results, please see Table 3 and Fig. 6. Fig. 6 is the histogram of the effects of the freeze-drying process on the anti-inflammatory effect of the product.

**Table 3**

| Group | Average inflammatory factor release level (pg/mL) |
|---|---|
| Untreated group | 9 |
| LPS control group | 396.7 |
| Non-freeze-drying group | 382.9 |
| Freeze-drying group | 79.3 |

The experimental results in Fig. 6 showed that the average inflammatory factor release level in the untreated group was 9 pg/mL; the average inflammatory factor release level in the LPS control group was 396.7 pg/mL; the average inflammatory factor release level in the non-freeze-drying group was 382.9 pg/mL; the average inflammatory factor release level in the freeze-drying group was 79.3 pg/mL. Compared with the non-freeze-drying group, the inflammatory factors release level in the freeze-drying group was significantly decreased (p<0.01). On the other hand, compared with the inflammatory factors release level in the non-freeze-drying group (382.9 pg/mL), the inflammatory factors release level in the freeze-drying group was significantly reduced to 79.3 pg/mL (from 100% to 21%; calculated as: 79.3÷382.9×100%=21%) (p>0.01). From the above experiments, it can be seen that the freeze-drying step in the present disclosure was an important technical feature and had unexpected effects.

The inventor expects that after inducing an inflammatory response in other human tissues with stimulants (e.g., LPS), the platelet-derived exosome dry powder obtained by the freeze-drying step in the present disclosure can also inhibit the induced inflammatory response, thereby alleviating inflammation of other tissues.

The above description is only a preferred embodiment of the present invention and is not intended to limit the scope of the patent application of the present invention. Therefore, any other changes or modifications made without departing from the spirit disclosed by the present invention are hereby deemed to be binding upon the patent application. All of them should be included in the scope of the present disclosure.

### References

| Serial number | References |
|---|---|
| 1 | Ian A. Darby and Tim D. Hewitson, 2007. Fibroblast Differentiation in Wound Healing and Fibrosis. International Review of Cytology, Vol. 257. |
| 2 | Chadanat Noonin and Visith Thongboonkerd, 2021. Exosome-inflammasome crosstalk and their roles in inflammatory responses. Theranostics 11(9): 4436-4451. |
| 3 | Laura M. Doyle and Michael Zhuo Wang. Overview of Extracellular Vesicles, Their Origin, Composition, Purpose, and Methods for Exosome Isolation and Analysis. Cells 2019, 8, 727. |
| 4 | Yi Zhang et al., Exosome: A Review of Its Classification, Isolation Techniques, Storage, Diagnostic and Targeted Therapy Applications. International Journal of Nanomedicine 2020:15 6917 - 6934 |

## Claims

1. A method for preparing a purified platelet-derived exosome dry powder, comprising:
(a) taking one unit of a solution derived from blood and containing platelets, and performing a process of purifying platelets to obtain a pure platelet solution; wherein step (a) comprises:
(a1) taking one unit of a solution derived from blood and containing platelets;
(a2) performing a first sedimentation process to separate the solution derived from blood and containing platelets into a plasma layer and a blood cell layer;
(a3) removing the blood cell layer to obtain a plasma layer solution;
(a4) performing a second sedimentation process to cause platelets to settle and form a pellet;
(a5) removing a supernatant and mixing the pellet with a solvent to obtain the pure platelet solution;
(b) performing an activation procedure to activate platelets in the pure platelet solution and release exosomes to obtain an activated pure platelet solution; wherein the activation procedure is a co-treatment procedure with collagen and thrombin;
(c) performing a purification process to obtain a purified platelet-derived exosome solution; and
(d) performing a drying process on the purified platelet-derived exosome solution to obtain a purified platelet-derived exosome dry powder containing a plurality of platelet-derived exosomes.

2. The method of claim 1, wherein the drying process is a freeze-drying process.

3. The method of claim 1, wherein step (a3) comprises a process of removing white blood cells so that a number of white blood cells per milliliter (mL) of the pure platelet solution is less than 10⁶.

4. The method of claim 1, wherein step (a5) comprises a process of removing plasma so that a concentration of albumin in the pure platelet solution is less than 3g/dL; or step (a5) comprises a process of removing plasma so that a concentration of globulin in the pure platelet solution is less than 2g/dL; or step (a5) comprises a process of removing plasma so that a concentration of fibrinogen in the pure platelet solution is less than 100µg/mL.

5. The method of claim 1, wherein the solution derived from blood and containing platelets is whole blood, and the blood cell layer comprises a buffy coat layer and a red blood cell layer.

6. The method of claim 1, wherein the activation procedure comprises:
(b1') mixing collagen and thrombin with the pure platelet solution; and (b2') vortexing at room temperature for 30 minutes.

7. The method of claim 1, wherein the activation procedure comprises:
(b1) mixing collagen and thrombin with the pure platelet solution to obtain a mixed solution, wherein a concentration of collagen in the mixed solution is 5-20µg/mL, and a concentration of thrombin in the mixed solution is 0.1-2U/mL; and
(b2) vortexing at room temperature for 30 minutes.

8. The method of claim 1, wherein the activation procedure comprises:
(b1') mixing collagen and thrombin with the pure platelet solution to obtain a mixed solution, wherein a concentration of collagen in the mixed solution reaches 7-12µg/mL; a concentration of thrombin reaches 0.5 -1.5U/mL;
(b2') vortexing at room temperature for 30 minutes.

9. The method of claim 2, wherein the freeze-drying process comprises lowering a temperature to less than or equal to -35°C and lowering a pressure to less than or equal to 80mTorr.

10. The method of claim 1, wherein the solution derived from blood and containing platelets is whole blood, apheresis platelet, leukocytes-reduced platelets apheresis, platelet-rich plasma (PRP) or any combination thereof; or the solution derived from blood and containing platelets is derived from an autologous blood sample, an allogeneic blood sample, a mammalian blood sample or a combination thereof.

11. The method of claim 1, wherein in step (a5), the solvent is sterile saline, water for injection, 0.45% NaCl, 4.5% hypertonic saline, sterile warm spring water or isotonic saline; or in step (a5), a platelet concentration in the pure platelet solution is 1×10⁹/mL to 10×10⁹/mL.

12. The method of claim 1, wherein the purification process comprises: (c1) performing a centrifugation process to remove most of platelet-associated structures; and (c2) performing a filter membrane filtration process to completely remove platelet-associated structures, wherein the filter membrane filtration process in step (c2) utilizes a filter membrane with a pore size of less than or equal to 0.45 µm to filter to obtain the purified platelet-derived exosome solution.

13. A purified platelet-derived exosome dry powder prepared by a preparation method, the preparation method comprising:
(a) taking one unit of a solution derived from blood and containing platelets, and performing a process of purifying platelets to obtain a pure platelet solution; wherein step (a) comprises:
(a1) taking one unit of a solution derived from blood and containing platelets;
(a2) performing a first sedimentation process to separate the solution derived from blood and containing platelets into a plasma layer and a blood cell layer;
(a3) removing the blood cell layer to obtain a plasma layer solution;
(a4) performing a second sedimentation process to cause platelets to settle and form a pellet;
(a5) removing a supernatant and mixing the pellet with a solvent to obtain the pure platelet solution;
(b) performing an activation procedure to activate platelets in the pure platelet solution and release exosomes to obtain an activated pure platelet solution; wherein the activation procedure is a co-treatment procedure with collagen and thrombin;
(c) performing a purification process to obtain a purified platelet-derived exosome solution; and
(d) performing a drying process on the purified platelet-derived exosome solution to obtain a purified platelet-derived exosome dry powder containing a plurality of platelet-derived exosomes.

14. The purified platelet-derived exosome dry powder of claim 13, wherein the drying process is a freeze-drying process.

15. The purified platelet-derived exosome dry powder of claim 13, wherein the activation procedure comprises:
(b1') mixing collagen and thrombin with the pure platelet solution to obtain a mixed solution, wherein a concentration of collagen in the mixed solution is 5-20µg/mL, and a concentration of thrombin in the mixed solution is 0.1-2U/mL; and
(b2') vortexing at room temperature for 30 minutes.

16. The purified platelet-derived exosome dry powder of any of claims 13 to 15, wherein in the purified platelet-derived exosome dry powder, a particle diameter of the platelet-derived exosomes is 20-150nm, and the platelet-derived exosomes comprise an exosome marker and a platelet marker;
wherein the exosome marker is at least one of CD9, CD63, and CD81 or any combination thereof, and the platelet marker is at least one of CD41 and CD42b or a combination thereof; wherein
(1) per gram (g) of the purified platelet-derived exosome dry powder contains at least 1×10¹⁰ platelet-derived exosomes; or
(2) per gram (g) of the purified platelet-derived exosome dry powder contains 1×10¹¹ to 1×10¹⁵ platelet-derived exosomes.

17. The purified platelet-derived exosome dry powder of any of claims 13 to 15, wherein
(1) per gram (g) of the purified platelet-derived exosome dry powder contains at least 50µg of microRNA; or
(2) a content of microRNA per gram (g) of the purified platelet-derived exosome dry powder is 100µg to 2,500µg.

18. The purified platelet-derived exosome dry powder of any of claims 13 to 15, wherein
(1) a content of PDGF-BB per gram (g) of the purified platelet-derived exosome dry powder is 0.01ng to 2,000ng; or
(2) a content of VEGF per gram (g) of the purified platelet-derived exosome dry powder is 50pg to 100,000pg; or
(3) a content of IGF per gram (g) of the purified platelet-derived exosome dry powder is 1pg to 3,000pg; or
(4) a content of TGF-β1 per gram (g) of the purified platelet-derived exosome dry powder is 50ng to 20,000ng; or
(5) a content of EGF per gram (g) of the purified platelet-derived exosome dry powder is 0.1ng to 200ng.

19. The purified platelet-derived exosome dry powder of any of claims 13 to 15, wherein
(1) no white blood cell-derived exosome is detected per gram of the purified platelet-derived exosome dry powder; or
(2) a number of white blood cell-derived exosomes per gram of the purified platelet-derived exosome dry powder is less than or equal to 1×10¹⁴;
wherein the white blood cell-derived exosome comprises a white blood cell marker; the white blood cell marker is CD45.

20. The purified platelet-derived exosome dry powder of any of claims 13 to 15, wherein
(1) no red blood cell-derived exosome is detected per gram of the purified platelet-derived exosome dry powder; or
(2) a number of red blood cell-derived exosomes per gram of the purified platelet-derived exosome dry powder is less than or equal to 1×10¹⁴;
wherein the red blood cell-derived exosome comprises a red blood cell marker; the red blood cell marker is at least one of CD235ar and Annexin V or a combination thereof.

21. The purified platelet-derived exosome dry powder of any of claims 13 to 15, wherein
(1) no exosomes other than the platelet-derived exosomes are detected per gram of the purified platelet-derived exosome dry powder; or
(2) a number of exosomes other than the platelet-derived exosomes per gram of the purified platelet-derived exosome dry powder is less than or equal to 1×10¹⁴.

22. Use of the purified platelet-derived exosome dry powder of any of claims 13 to 15 in the manufacture of a pharmaceutical composition or health composition for administrating an effective dose to a site of an individual to relieve a degree of inflammation or injury of the site of the individual.

23. The use of claim 22, wherein the purified platelet-derived exosome dry powder is used in the manufacture of the pharmaceutical composition or health composition to relieve the degree of inflammation or injury in a respiratory tract, skin, muscle, tendon, bone, joint or ligament of the individual.

24. Use of the purified platelet-derived exosome dry powder of any of claims 13 to 15 in the manufacture of a pharmaceutical composition or health composition for enhancing a migration ability of dermal fibroblasts into a skin site.

25. The use of claim 24, wherein the skin site is a skin lesion site.

26. Use of the purified platelet-derived exosome dry powder of any of claims 13 to 15 in the manufacture of a pharmaceutical composition or health composition for enhancing an expression quantity of collagen in dermal fibroblasts.

27. A method for preparing a purified platelet-derived exosome dry powder, comprising:
(a) taking one unit of a solution derived from blood and containing platelets, and performing a process of purifying platelets to obtain a pure platelet solution;
wherein step (a) comprises:
(a1) taking one unit of a solution derived from blood and containing platelets;
(a2) performing a first sedimentation process to separate the solution derived from blood and containing platelets into a plasma layer and a blood cell layer;
(a3) removing the blood cell layer to obtain a plasma layer solution;
(a4) performing a second sedimentation process to cause platelets to settle and form a pellet;
(a5) removing a supernatant and mixing the pellet with a solvent to obtain the pure platelet solution;
(b) performing an activation procedure to activate platelets in the pure platelet solution and release exosomes to obtain an activated pure platelet solution; wherein the activation procedure is a co-treatment procedure with collagen and adenosine diphosphate (ADP);
(c) performing a purification process to obtain a purified platelet-derived exosome solution; and
(d) performing a drying process on the purified platelet-derived exosome solution to obtain a purified platelet-derived exosome dry powder containing a plurality of platelet-derived exosomes.

28. The method of claim 27, wherein the activation procedure comprises:
(b 1) mixing collagen and ADP with the pure platelet solution to obtain a mixed solution, wherein a concentration of collagen in the mixed solution is 5-20µg/mL, and a concentration of ADP in the mixed solution is 40-80µM; and
(b2) vortexing at room temperature for 30 minutes.
